# FASCICULE DE BREVET EUROPEEN

(11) **EP 1 960 335 B1**
(45) Date de publication et mention de la délivrance du brevet: **14.04.2010**
(21) Numéro de dépôt: 06841985.2
(22) Date de dépôt: 18.10.2006
(51) Int. Cl.: C07C 17/20

(54) **PROCEDE DE FABRICATION DU PENTAFLUOROETHANE**
VERFAHREN ZUR HERSTELLUNG VON PENTAFLUORETHAN
PENTAFLUOROETHANE PRODUCING METHOD

(30) Priorité: 06.12.2005 FR 0512338
(43) Date de publication de la demande: 27.08.2008
(73) Titulaire: ARKEMA FRANCE, 92700 Colombes (FR)
(72) Inventeur: BOUSSAND, Béatrice, F-69110 Sainte Foy les Lyon (FR); DEVIC, Michel, F-69110 Sainte Foy les Lyon (FR)
(74) Mandataire: Dang, Doris
(86) Numéro de dépôt international: PCT/FR2006/051052
(87) Numéro de publication internationale: WO 2007/066021

(56) Documents cités:
- EP-A- 1 038 858
- WO-A-2004/018396

## Description

La présente invention concerne un procédé continu de fluoration du perchloroéthylène et a plus particulièrement pour objet la fluoration du perchloroéthylène en phase gazeuse avec de l'acide fluorhydrique (HF) en présence d'un catalyseur pour donner, comme produit majoritaire, le pentafluoroéthane.

La réaction de fluoration du perchloroéthylène avec de l'HF en phase gazeuse en présence d'un catalyseur est connue. Elle conduit en général à la formation du 2,2-dichloro-1,1,1-trifluoroéthane (123), 2-chloro-1,1,1,2-tetrafluoroéthane (124) et pentafluoroéthane (125) avec le 123 comme produit majoritaire.

Ces composés (globalement désignés ci-après par l'expression « série 120 ») peuvent être utilisés soit comme substituts des chlorofluorocarbures (CFC) dans les domaines des mousses (agents gonflants et isolants), des aérosols (agents propulseurs) ou dans la réfrigération, soit comme intermédiaires de synthèse de ces substituts. On recherche actuellement des procédés performants pour la production industrielle du pentafluoroéthane.

Le document WO 92/16479 divulgue un procédé de fabrication du 2,2-dichloro-1,1,1-trifluoroéthane (123), 2-chloro-1,1,1,2-tetrafluoroéthane (124) et pentafluoroéthane (125) en faisant réagir du perchloroéthylène (PER) avec de l'HF en phase gazeuse en présence d'un catalyseur contenant le fluorure de Zn supporté sur de l'alumine fluoré à une température comprise entre 250 et 450°C et avec un temps de contact compris entre 0,1 et 60 sec. Ce document enseigne d'utiliser de préférence un ratio molaire HF/PER compris entre 3 et 10. Le document WO 2004/018396 préconise in ratio molaire HF/PER inférieur à 15.

Pour obtenir le pentafluoroéthane comme produit majoritaire, on recommande d'opérer en deux étapes. Ainsi le document EP 811592 fournit un procédé en deux étapes selon lequel on fait réagir du PER avec de l'HF en phase liquide en présence d'un catalyseur et à une température comprise entre 60 et 150°C pour donner du 123 et/ou 1,1,2-trichloro-2,2-difluoroéthane (122) et ensuite on fait réagir en phase vapeur le 123 et/ou 122, formé dans la première étape, avec de l'HF en présence d'un catalyseur et à une température comprise entre 250 et 450°C.

Par ailleurs, ce document enseigne que la réaction de fluoration du perchloroéthylène en pentafluoroéthane étant une réaction fortement exothermique (28 Kcal/mol), sa mise en oeuvre en une seule étape pose de nombreux problèmes : la réaction est difficile à contrôler, le catalyseur se dégrade et il y a formation de sous-produits en quantité importante.

La Société déposante a mis au point un procédé continu de fabrication du pentafluoroéthane en une seule étape en faisant réagir du perchloroéthylène avec de l'HF en phase gazeuse et ne présentant pas les inconvénients précités.

La présente invention fournit donc un procédé de fabrication du pentafluoroéthane en une seule étape en faisant réagir en phase gazeuse le perchloroéthylène avec de l'HF en présence d'un catalyseur caractérisé en ce que l'on opère à une température comprise entre 280 et 430°C et avec un ratio molaire HF/PER supérieur ou égal à 20.

Le procédé selon la présente invention est de préférence mis en oeuvre à une température comprise entre 300 et 400°C et avantageusement comprise entre 340 et 370°C.

Le ratio molaire HF/PER est de préférence compris entre 20 et 60 et avantageusement compris entre 20 et 30.

Selon un mode préféré de l'invention, le ratio molaire HF/PER représente un nombre supérieur à 20 et inférieur ou égal à 60.

Selon un mode particulièrement préféré de l'invention, le ratio molaire HF/PER représente un nombre supérieur à 20 et inférieur ou égal à 30.

De manière surprenante, les inventeurs ont observé qu'à partir d'un ratio molaire HF/PER de 20, le débit d'HF à l'entrée du réacteur par unité de 125 produit commence à décroître. Les inventeurs ont également observé que le débit d'HF à l'entrée du réacteur par unité de 125 produit est optimal dans l'intervalle préféré de ratio molaire HF/PER. En outre, ils ont observé que dans l'intervalle préféré de ratio molaire HF/PER, la quantité de 125 produit par unité de catalyseur mis en jeu est également optimale.

Dans le procédé selon la présente invention le temps de contact, calculé comme étant le temps de passage des gaz (dans les conditions réactionnelles) à travers le volume de catalyseur est compris entre 2 et 50 sec., de préférence compris entre 3 et 25 sec.

Le procédé peut être mis en oeuvre à une pression inférieure ou égale à 5 bar absolu.

De préférence, on opère à une pression comprise entre 1 et 3 bars absolu.

Tout catalyseur de fluoration peut convenir au procédé de la présente invention. Le catalyseur utilisé comprend de préférence les oxydes, halogénures, oxyhalogénures ou sels minéraux du chrome, d'aluminium, de cobalt, de manganèse, de nickel, de fer ou de zinc, et pouvant être supporté. A titre d'exemple, on peut citer l'alumine, le fluorure d'aluminium, ou l'oxyfluorure d'aluminium comme support.

On utilise de préférence un catalyseur à base d'oxyde de chrome (Cr₂O₃) incluant éventuellement un autre métal de degré d'oxydation supérieur à zéro et sélectionné parmi le Ni, Co, Mn et Zn. Avantageusement ce catalyseur peut être supporté sur de l'alumine, de l'aluminium fluoré ou de l'oxyfluorure d'aluminium.

Un catalyseur mixte composé d'oxydes, d'halogénures et/ou d'oxyhalogénure de nickel et de chrome déposés sur un support constitué de fluorure d'aluminium ou d'un mélange de fluorures d'aluminium et d'alumine convient tout particulièrement au procédé de la présente invention.

Ce catalyseur peut être préparé de façon connue en soi à partir d'une alumine activée. Celle-ci peut dans une première étape être transformée en fluorure d'aluminium ou en mélange de fluorure d'aluminium et d'alumine, par fluoration à l'aide d'acide fluorhydrique en présence éventuellement d'air ou d'un inerte tel que l'azote à une température en général comprise entre 200 et 450°C, de préférence entre 250 et 400°C. Le support est ensuite imprégné à l'aide de solutions aqueuses de sels de chrome et de nickel ou à l'aide de solutions aqueuses d'acide chromique, dé sel de nickel et d'un réducteur de chrome comme le méthanol.

Lorsque l'on utilise l'acide chromique (CrO₃) comme précurseur du chrome, on peut réduire ce chrome par tout moyen connu de l'homme de l'art sous réserve que la technique utilisée ne nuise pas aux propriétés du catalyseur et donc à son activité. Le réducteur préféré est le méthanol.

Comme sels de chrome et de nickel, on préfère utiliser les chlorures, mais on peut aussi employer d'autres sels tels que, par exemple, les oxalates, formiates, acétates, nitrates et sulfates ou le bichromate de nickel pourvu que ces sels soient solubles dans la quantité d'eau susceptible d'être absorbée par le support.

Le catalyseur mixte utilisé dans le procédé selon l'invention peut aussi être préparé par imprégnation directe de l'alumine par des solutions des composés de chrome et de nickel ci-dessus mentionnées. Dans ce cas, la transformation d'au moins une partie de l'alumine en fluorure d'aluminium s'effectue lors de l'étape d'activation du catalyseur.

Les alumines activées à utiliser pour la préparation du catalyseur mixte sont des produits bien connus, disponibles dans le commerce. Elles sont généralement préparées par calcination d'hydrates d'alumine à une température comprise entre 300 et 800°C. Les alumines activées, utilisables dans le cadre de la présente invention, peuvent contenir des teneurs importantes (jusqu'à 100 ppm) de sodium sans que cela nuise à l'activité catalytique.

Le catalyseur mixte peut contenir en masse de 0,5 à 20 % de chrome et de 0,5 à 20 % de nickel et, de préférence, entre 2 et 10% de chacun des métaux dans un rapport atomique nickel/chrome compris entre 0,5 et 5, de préférence voisin de 1.

Avant utilisation dans la réaction de fluoration du perchloroéthylène, le solide catalytique est préalablement soumis à une opération d'activation.

Ce traitement réalisé soit « in situ » (dans le réacteur de fluoration) soit dans un appareillage adéquat comprend généralement les étapes suivantes :
- séchage à basse température en présence d'air ou d'azote,
- séchage à haute température (250 à 450°C, de préférence 300 à 350°C) sous azote ou sous air,
- fluoration à basse température (180 à 350°C) au moyen d'un mélange d'acide fluorhydrique et d'azote, en contrôlant la teneur en HF de façon que la température ne dépasse pas 350°C et finition sous courant d'acide fluorhydrique pur ou dilué par de l'azote à une température pouvant aller jusqu'à 450°C.

Selon une variante du procédé, les produits de la réaction après séparation du 125 peuvent être recyclés.

Le procédé selon la présente invention a l'avantage d'augmenter la proportion en produits très fluorés (124, 125) dans le mélange en sortie du réacteur ce qui en mode recyclage permet de réduire la quantité de produits sous-fluorés à recycler, certains de ces produits étant à l'origine d'une désactivation du catalyseur par cokage.

Bien que cela ne soit pas nécessaire pour la réaction de fluoration, il peut être judicieux d'introduire avec les réactifs de l'oxygène à faible teneur. Cette teneur peut varier selon les conditions opératoires entre 0,02 et 1 % molaire par rapport au mélange gazeux entrant dans le réacteur.

Le procédé selon la présente invention peut être mis enoeuvre dans un réacteur construit à partir des matériaux résistant à la corrosion par exemple HASTELLOY, INCONEL et MONEL. Tout type de réacteurs peut convenir : par exemple réacteur multi-tubulaire, réacteur adiabatique.

### PARTIE EXPERIMENTALE

### Préparation du catalyseur

Dans un évaporateur rotatif, on place 343 g d'un support obtenu dans une étape précédente par fluoration d'alumine GRACE HSA en lit fixe vers 280°C à l'aide d'air et d'acide fluorhydrique (concentration volumique de 5 à 10 % de cet acide dans l'air). L'alumine GRACE HSA de départ présente les caractéristiques physicochimiques suivantes :
- formé : billes de 0,5-2 mm de diamètre
- surface BET : 220 m²/g
- volume poreux : 1,3 cm³/g

On prépare par ailleurs deux solutions aqueuses séparées :
(a) solution chromique additionnée de chlorure de nickel contenant :

| | |
|---|---|
| - anhydride chromique | 55 g |
| - chlorure de nickel hexahydraté | 130 g |
| - eau | 63 g |

(b) Solution méthanolique contenant :

| | |
|---|---|
| - méthanol | 81 g |
| - eau | 8 g |

Ces deux solutions sont introduites simultanément à une température de 40°C sous pression atmosphérique et en 2 heures environ, sur le support en agitation. Après une étape de maturation sous azote, le catalyseur est séché sous azote, puis sous vide à 65°C puis vers 90°C pendant 6 heures.

On charge 500 g de solide imprégné dans un réacteur tubulaire en inconel. Le catalyseur est tout d'abord séché sous balayage d'azote à basse température puis jusqu'à 320°C, à pression atmosphérique. Il est ensuite fluoré en présence d'un mélange acide fluorhydrique / azote (concentration volumique de 5 à 10 % de cet acide dans l'azote) à 320°C puis jusqu'à 390°C. L'alimentation d'HF est alors coupée. Le balayage à l'azote est maintenu pendant 15 minutes à 390°C puis le catalyseur est refroidi jusqu'à 60°C sous balayage d'azote.

Les exemples ci-dessous ont été réalisés à l'aide d'un pilote de fluoration en phase gaz en continu. Ce pilote comprend un réacteur constitué d'un tube en inconel d'un diamètre interne de 22,5 mm et d'une longueur de 500 mm, placé verticalement dans un four électrique tubulaire. Une gaine en inconel de 1/8 de pouce (3,175mm) est placée coaxialement au centre du tube et un thermocouple mobile permet de relever le profil de température le long du réacteur.

Le lit catalytique est constitué d'une couche inférieure de 180 mm de corindon puis d'une couche de catalyseur de 120 mm (volume : 48 cm³, masse : 42,9 g) et d'une couche supérieure de 60 mm de corindon.

Le catalyseur est d'abord séché à pression atmosphérique puis il est activé par passage d'un débit d'HF de 0,286 mol/h à 355°C pendant 24 h. Un débit de 12 I/h d'azote est ajouté pendant la première heure de l'activation du catalyseur.

Les réactifs sont ensuite introduits en continu à l'extrémité supérieure du réacteur et préchauffés à la température du four à travers la couche supérieure de corindon, les produits gazeux de la réaction sortent à l'extrémité inférieure du réacteur à travers une vanne de régulation de pression, le flux gazeux sortant de la vanne est analysé par chromatographie phase gaz.

Les caractéristiques du catalyseur après activation sont les suivantes :
- surface BET : 40 m²/g
- volume poreux : 0,4 cm³/g
- composition chimique pondérale :
   ■ Al : 25 %
   ■ F : 58 %
   ■ Cr : 5,3 %
   ■ Ni : 6,4 %

Une même quantité de 42,9 g est utilisée pour tous les essais du tableau 1.

| | Ex 1 | Ex 2 | Ex 3 | Ex 4 | Ex 5 | Ex 6 | Ex 7 |
|---|---|---|---|---|---|---|---|
| Température (maximale) du point chaud du catalyseur °C | 359 | 358 | 358 | 359 | 360 | 360 | 360 |
| Rapport molaire HF/PER | 10 | 20 | 25 | 30 | 25 | 26 | 45 |
| Débit HF à l'entrée du réacteur mmol/h | 630 | 1014 | 658 | 528 | 617 | 640 | 652 |
| Débit PER à l'entrée du réacteur mmol/h | 62,9 | 50,9 | 26,32 | 17,8 | 24,14 | 24,25 + 9,52 (124) | 14,39 + 6,45 (124) + 1,34 (123) |
| Pression à l'entrée du réacteur (bar absolu) | 1 | 1 | 1 | 1 | 2 | 2 | 2 |
| TTG PER % | 63,3 | 70,9 | 88,7 | 93,5 | 97,9 | 99 | 100 |
| Composition du mélange organique en sortie du réacteur (% mol) | | | | | | | |
| 125 | 19,2 | 33,2 | 50,5 | 62 | 47,1 | 68 | 70 |
| 124 | 36,5 | 34,7 | 29,2 | 23,7 | 32,3 | 21,8 | 19,4 |
| 123 | 29,6 | 20,6 | 12,6 | 8,3 | 12,5 | 5,76 | 4,8 |
| Débit de 125 à la sortie du réacteur mmol/h | 7,65 | 11,96 | 11,8 | 10,32 | 11,37 | 22,95 | 15,52 |
| Productivité en 125 en g/h/l de catalyseur | 19,1 | 29,9 | 29,5 | 25,8 | 28,4 | 57,3 | 38,8 |
| Flux d'HF (Kg) à l'entrée du réacteur par Kg de 125 produit | 13,73 | 14,13 | 9,27 | 8,53 | 9,04 | 4,65 | 7,0 |

| | | | | | | | |
|---|---|---|---|---|---|---|---|
| TTG : taux de transformation (conversion). | | | | | | | |

## Revendications

1. Procédé de fabrication du pentafluoroéthane en une seule étape en faisant réagir en phase gazeuse le perchloroéthylène avec de l'HF en présence d'un catalyseur **caractérisé en ce que** l'on opère à une température comprise entre 280 et 430°C et avec un ratio molaire HF/PER supérieur ou égal à 20.

2. Procédé selon la revendication 1 **caractérisé en ce que** la température est comprise entre 300 et 400°C et de préférence comprise entre 340 et 370°C.

3. Procédé selon la revendication 1 ou 2 **caractérisé en ce que** le ratio molaire HF/PER est compris entre 20 et 60 et de préférence compris entre 20 et 30.

4. Procédé selon l'une quelconque des revendications précédentes **caractérisé en ce que** l'on opère à une pression inférieure ou égale à 5 bar absolu, de préférence à une pression comprise entre 1 et 3 bars absolu.

5. Procédé selon l'une quelconque des revendications précédentes **caractérisé en ce que** le catalyseur comprend des oxydes, halogénures, oxyhalogénures ou sels minéraux du chrome, d'aluminium, de cobalt, de manganèse, de nickel, de fer ou de zinc.

6. Procédé selon l'une quelconque des revendications précédentes **caractérisé en ce que** le catalyseur à base d'oxyde de chrome (Cr₂O₃) incluant éventuellement un autre métal de degré d'oxydation supérieur à zéro et sélectionné parmi le Ni, Co, Mn et Zn.

7. Procédé selon l'une quelconque des revendications précédentes **caractérisé en ce que** le catalyseur mixte est composé d'oxydes, d'halogénures et/ou d'oxyhalogénure de nickel et de chrome.

8. Procédé l'une quelconque des revendications précédentes **caractérisé en ce que** le catalyseur est supporté.

9. Procédé selon la revendication 8 **caractérisé en ce que** le catalyseur est supporté sur l'alumine, le fluorure d'aluminium, ou l'oxyfluorure d'aluminium.

## Claims

1. Process for the manufacture of pentafluoroethane in a single stage by reacting perchloroethylene with HF in the gas phase in the presence of a catalyst, **characterized in that** the process is carried out at a temperature of between 280 and 430°C and with an HF/PER molar ratio of greater than or equal to 20.

2. Process according to Claim 1, **characterized in that** the temperature is between 300 and 400°C and preferably between 340 and 370°C.

3. Process according to Claim 1 or 2, **characterized in that** the HF/PER molar ratio is between 20 and 60 and preferably between 20 and 30.

4. Process according to any one of the preceding claims, **characterized in that** the process is carried out at a pressure of less than or equal to 5 bar absolute, preferably at a pressure of between 1 and 3 bar absolute.

5. Process according to any one of the preceding claims, **characterized in that** the catalyst comprises oxides, halides, oxyhalides or inorganic salts of chromium, aluminium, cobalt, manganese, nickel, iron or zinc.

6. Process according to any one of the preceding claims, **characterized in that** the catalyst is based on chromium oxide (Cr₂O₃) optionally including another metal at an oxidation state of greater than zero selected from Ni, Co, Mn and Zn.

7. Process according to any one of the preceding claims, **characterized in that** the mixed catalyst is composed of oxides, halides and/or oxyhalides of nickel and chromium.

8. Process according to any one of the preceding claims, **characterized in that** the catalyst is supported.

9. Process according to Claim 8, **characterized in that** the catalyst is supported on alumina, aluminium fluoride or aluminium oxyfluoride.

## Patentansprüche

1. Verfahren zur Herstellung von Pentafluorethan in einem einzigen Schritt, bei dem das Perchlorethylen in der Gasphase in Gegenwart eines Katalysators mit HF reagieren gelassen wird, **dadurch gekennzeichnet, dass** bei einer Temperatur im Bereich von 280 bis 430 °C und mit einem Molverhältnis HF/PER von größer oder gleich 20 gearbeitet wird.

2. Verfahren nach Anspruch 1, **dadurch gekennzeichnet, dass** die Temperatur im Bereich von 300 bis 400 °C und vorzugsweise im Bereich von 340 bis 370 °C liegt.

3. Verfahren nach Anspruch 1 oder 2, **dadurch gekennzeichnet, dass** das Molverhältnis HF/PER im Bereich von 20 bis 60 und vorzugsweise im Bereich von 20 bis 30 liegt.

4. Verfahren nach einem der vorhergehenden Ansprüche, **dadurch gekennzeichnet, dass** bei einem Druck von kleiner oder gleich 5 bar absolut und vorzugsweise bei einem Druck im Bereich von 1 bis 3 bar absolut gearbeitet wird.

5. Verfahren nach einem der vorhergehenden Ansprüche, **dadurch gekennzeichnet, dass** der Katalysator Oxide, Halogenide, Halogenidoxide oder anorganische Salze von Chrom, Aluminium, Cobalt, Mangan, Nickel, Eisen oder Zink umfasst.

6. Verfahren nach einem der vorhergehenden Ansprüche, **dadurch gekennzeichnet, dass** es sich um einen Katalysator auf der Basis von Chromoxid (Cr₂O₃) handelt, der gegebenenfalls ein weiteres Metall mit einer Oxidationsstufe über Null aufweist, das unter Ni, Co, Mn und Zn ausgewählt ist.

7. Verfahren nach einem der vorhergehenden Ansprüche, **dadurch gekennzeichnet, dass** der gemischte Katalysator aus Oxiden, Halogeniden und/oder Halogenidoxiden von Nickel und Chrom gebildet ist.

8. Verfahren nach einem der vorhergehenden Ansprüche, **dadurch gekennzeichnet, dass** sich der Katalysator auf einem Träger befindet.

9. Verfahren nach Anspruch 8, **dadurch gekennzeichnet, dass** als Träger für den Katalysator Aluminiumoxid, Aluminiumfluorid oder Aluminiumfluoridoxid verwendet wird.
